# EUROPÄISCHE PATENTSCHRIFT

(11) **EP 0 450 423 B1**
(45) Veröffentlichungstag und Bekanntmachung des Hinweises auf die Patenterteilung: **29.01.1997**
(21) Anmeldenummer: 91104497.2
(22) Anmeldetag: 22.03.1991
(51) Int. Cl.: A61B 17/60, A61F 5/04

(54) **Vorrichtung zur Retention von Körperteilen mit lädierten Bändern und/oder mit lädierten Knochen**
Apparatus for the retention of body parts with injured ligaments and/or bones
Appareil pour la rétention de parties du corps avec ligaments et/ou os blessés

(30) Priorität: 27.03.1990 DE 4009722; 06.11.1990 DE 4035227
(43) Veröffentlichungstag der Anmeldung: 09.10.1991
(73) Patentinhaber: Clasbrummel, Bernhard, 44795 Bochum (DE)
(72) Erfinder: Clasbrummel, Bernhard, 44795 Bochum (DE)
(74) Vertreter: Witte, Alexander, Dr.-Ing.

(56) Entgegenhaltungen:
- EP-A- 0 128 115
- EP-A- 0 135 394
- EP-A- 0 420 430
- FR-A- 2 628 627
- BIOMEDIZINISCHE TECHNIK, Band 22, Nr. 12, Dezember 1977, Seiten 299-302, Berlin, DE; H. THOMA et al.: "Die Zugmessung bei Knochendistraktion: Methode und klinische Ergebnisse"
- IDEM
- SOVIET INVENTIONS ILLUSTRATED, Teil P/Q, Woche B43, 5. Dezember 1979, Klasse P31, Nr. K1686B/43, Derwent Publicatons Ltd, London, GB; & SU- A-644 469 (TRAUMATOLOGY ORTHOPAEDICS)

## Beschreibung

Die Erfindung betrifft eine Vorrichtung zur Retention von Körperteilen mit lädierten Bändern und/oder lädierten Knochen, mit zumindest einem Antrieb zum stimulierenden rhythmischen Hin- und Herbewegen der retentierten Körperteile in einem Läsionsbereich mit einer bestimmten Stimulationsamplitude und einer bestimmten Stimulationskraft.

Die Erfindung betrifft ferner ein Verfahren zur Retention von Körperteilen mit lädierten Bändern und/oder lädierten Knochen, wobei durch rhythmisches Hin- und Herbewegen der retentierten Körperteile im Läsionsbereich mit einer bestimmten Stimulationsamplitude und einer bestimmten Stimulationskraft stimuliert wird.

Eine Vorrichtung zur Retention von Körperteilen mit lädierten Knochen, nämlich mit Knochenfrakturen, ist aus dem Dokument "Controlled Mechanical Stimulation in the Treatment of Tibial Fractures", J. Kenwright et al., Clin. Orthop. and Related Research, 1989, 241, S. 36 bis 47, bekannt.

Bänderläsionen nach (Distorsions-) Trauma, mit und ohne Knochenbeteiligung, stellen in der Unfallchirurgie ein sehr häufiges Problem dar. Die Genesungsphase dauert je nach Schwere der Verletzung in der Regel mehrere Wochen bis Monate und ist sehr kostenintensiv (Krankenhauskosten und Arbeitsausfallskosten). Der Belastungsbeginn nach konservativer oder operativer Behandlung ist insbesondere von der Erfahrung des Therapeuten abhängig.

Die bekannte Vorrichtung zur Retention von Knochen ist als Fixateur Externe ausgebildet. Ein Fixateur Externe wird dazu verwendet, um nach einer Fraktur oder Osteotomie eines Knochens, insbesondere des in einem Bein oder Arm eines Patienten vorhandenen Knochens, die Knochenstücke oder -Fragmente mit Stiften zu fixieren, die von der Außenseite des Körpers her durch die Haut hindurch in die Knochenstücke eingesetzt, vorzugsweise eingeschraubt werden. Die Stifte sind mit außerhalb des Körpers gelegenen Haltern verbunden, die wiederum in einem Verstellelement aufgenommen sind. Am Fixateur ist ein Antrieb vorgesehen, der dafür sorgt, daß sich die beidseits der Frakturstelle angeordneten Stifte relativ zueinander hin- und herbewegen, wodurch auch die Knochenfragmente, in denen die Stifte eingebracht sind, hin- und herbewegen. Die Amplituden liegen dabei im Millimeterbereich. Es wurde festgestellt, daß durch eine derartige kontrollierte, mechanische Stimulation eine Verkürzung der Knochenheilungszeit erreicht werden kann. Der Antrieb ist dabei derart ausgestaltet, daß während der gesamten Behandlungszeit die Stimulation mit einer gleichbleibenden Stimulationsamplitude und mit einer gleichbleibenden Stimulationskraft durchgeführt wird.

Aus der Druckschrift FR-2 628 627 ist eine Vorrichtung zum Verlängern von Gliedmaßen bekannt.

Bei der Verlängerung von Gliedmaßen (Knochendistraktion) werden mittels eines Fixateurs Externe die absichtlich gebrochenen Knochenteile soweit auseinandergehalten, daß der Heilungsvorgang gerade noch einsetzen kann. Hat sich eine bestimmte Knochenmenge gebildet, so wird der Abstand zwischen den Knochenteilen verlängert. Dieser Vorgang wird so oft wiederholt, bis die gewünschte Verlängerung, die beim Bein bis zu 6 cm betragen kann, erreicht ist.

Der Abstand zwischen den Knochenteilen kann bei der bekannten Vorrichtung mittels einer Steuereinheit über einen Motor eingestellt werden. Es ist dabei möglich, den Abstand schrittweise bzw. kontinuierlich zu vergrößern.

Aus der Druckschrift FR-2 628 627 ist desweiteren ein Druckmesser bekannt, der die Gegenkraft der Haut, der Sehnen, der Nerven, der Blutgefäße und der Muskeln, die ebenfalls mehrere Zentimeter verlängert werden müssen, erfaßt.

Aus der Veröffentlichung BIOMEDIZINISCHE TECHNIK, Band 22, Nr. 12, Dez. 1977, H. Thoma et al. "Die Zugmessung bei Knochendistraktion: Methode und klinische Ergebnisse" ist eine Vorrichtung zum Messen des bei der Verlängerung von Gliedmaßen (Distraktion) auftretenden Zuges bekannt. Die Messung des Distraktionszuges wird dabei pneumatisch durchgeführt. Mit dieser Vorrichtung kann ein Zug-Weg-Diagramm erstellt werden.

Aufgabe der vorliegenden Erfindung ist, eine Vorrichtung und ein Verfahren der eingangs genannten Art derart weiterzuentwickeln, daß eine weitere Verkürzung der Heilungszeit erreicht werden kann, insbesondere auch bei lädierten Bändern.

Erfindungsgemäß wird die Aufgabe bei einer Vorrichtung durch einen Regelkreis gelöst, mit einem Meßglied, das die Festigkeit im Läsionsbereich erfaßt, mit einer Regeleinheit, die die vom Meßglied erfaßten Festigkeitsdaten als Basis für ein Regelsignal heranzieht, und mit einem Stellglied, das die Stimulationsamplitude und die Stimulationskraft während eines Heilungsvorganges in Abhängigkeit vom Regelsignal einstellt.

Erfindungsgemäß wird die Aufgabe bei einem Verfahren dadurch gelöst, daß die Festigkeitsentwicklung im Läsionsbereich gemessen wird, daß daraus ein Regelsignal bestimmt wird, und daß in Abhängigkeit der erfaßten Daten der Festigkeitsentwicklung die Stimulationskraft und die Stimulationsamplitude geregelt wird.

Zu Beginn eines Heilungsvorganges einer Knochenfraktur bildet sich an den gegenüberliegenden Knochenbruchstellen zunächst relativ weiches, nach und nach verknorpelndes und später verknöcherndes Regenerationsgewebe. In diesem anfänglichen Heilungsstadium kann eine Beschleunigung des Heilungsprozesses dadurch erreicht werden, daß mit einer relativ großen Stimulationsamplitude und einer relativ geringen Stimulationskraft stimuliert wird. Bei fortschreitendem Heilungsprozeß verfestigt sich die Knochensubstanz Zwischen den zusammenwachsenden Bruchstellen immer mehr, wobei festgestellt wurde, daß eine Stimulierung mit geringer werdender Stimulationsamplitude und zunehmender Stimulationskraft eine erhebliche Verkürzung der Heilungszeit zur Folge hat. Der jetzt erfindungsgemäße vorgeschlagene Regelkreis regelt somit die Stimulationsamplitude und Stimulationskraft in Abhängigkeit von der Festigkeitsentwicklung während des Heilungsprozesses und ermöglicht daher die in dem jeweiligen Stadium des Heilungsprozesses optimale Stimulation zu ermöglichen. Dadurch wird eine erhebliche Verkürzung der Heilungszeit erreicht. Bei einem Heilungsprozeß von lädierten Bändern kann es wünschenswert sein, zunächst eine Stimulation mit einer relativ geringen Stimulationsamplitude durchzuführen, um beispielsweise frisch genähte Bänder nicht wieder auseinanderzureißen. Im Verlaufe des Heilungsprozesses ist dann von Vorteil, die Stimulationsamplitude und auch die Stumulationskraft zu steigern, um schon während des Heilungsprozesses die Dehnfähigkeit und Widerstandsfähigkeit des lädierten Bandes wieder herzustellen. Der Zeitverlauf eines Heilungsprozesses, beispielsweise bei einer Knochenfraktur, ist im wesentlichen von dem Allgemeinzustand, insbesondere durch das Alter des jeweiligen lebendem Individiums abhängig. Liegen Erfahrungswerte über den Heilungsprozeß bestimmter Altersgruppen vor, so kann der Regelkreis entsprechend dem Heilungsverhalten dieser Altersgruppe programmiert werden, so daß die Regelung der Stimulationsamplitude und der Stimulationskraft vorprogrammiert werden können.

In einer besonderen Ausgestaltung der Erfindung ist im Regelkreis ein Meßglied vorgesehen, das die Festigkeit im Läsionsbereich erfaßt, und die vom Meßglied erfaßten Festigkeitsdaten dienen als Basis für ein Regelsignal einer Regeleinheit.

Diese Maßnahme hat den Vorteil, daß die Regelung der Stimulationsamplitude und der Stimulationskraft in Abhängigkeit von dem jeweiligen Heilungsverlauf durchgeführt wird. Dadurch können zu frühzeitige und zu starke, heilungsstörende Belastungen als auch ein heilungsverzögerndes Abwarten der Anpassung vermieden werden. Es findet somit eine rückkopplungsgesteuerte, kontrollierte mechanische Stimulation (RKMS) statt, die über das Meßglied und die Regeleinheit den jeweils optimalen Regelzustand in einem bestimmten Abschnitt des Heilungsverlaufes ermöglicht. Dies kann vollautomatisch programmgesteuert erfolgen, so daß nach Anbringen des Meßgliedes der weitere Regelungsablauf der Stimulationsamplitude und Stimulationskraft vollautomatisch abläuft. Dies verkürzt nicht nur die Behandlungszeit und die damit für die Allgemeinheit bzw. die entsprechende Person verbundenen Kosten, sondern spart auch noch zusätzlich Kosten dahingehend, daß die Behandlungsvorgänge nicht laufend überwacht werden müssen. Es ist beispielsweise dann möglich, Patienten mit Knochenbrüchen schon nach relativ kurzer Zeit aus einem Krankenhaus zu entlassen und den Heilungsprozeß, vollautomatisch überwacht und optimal angepaßt, zu Hause ablaufen zu lassen.

In einer weiteren Ausgestaltung der Erfindung wird die Stimulationskraft während eines Stimulationsvorganges mittels eines Kraftwandlers in einem Kraft-Zeit-Profil stimuliert, das einem Kraft-Zeit-Profil eines typischen Bewegungsablaufes der retenierten Körperteile entspricht.

Diese Maßnahme hat den Vorteil, daß die Belastung der Körperteile bei der Stimulation dem Belastungsprofil möglicher Bewegungsabläufe angepaßt ist. So ist beispielsweise aus der Veröffentlichung "K. Willimczik, 1989, Biomechanik der Sportarten, Rowohlt, 8601, 145 bis 148", bekannt geworden, daß beispielsweise beim Gehen, Laufen und Sprinten charakteristische Kraft-Zeit-Verläufe in horizontaler und vertikaler Richtung stattfinden. Hat nun beispielsweise ein Sprint-Sportler, beispielsweise 100- oder 200-Meter-Läufer eine Knochenfraktur erlitten, so ist es mit der erfindungsgemäßen Vorrichtung möglich, während des Heilungsprozesses einen Vorgang zu stimulieren, der beispielsweise ein Kraft-Zeit-Profil aufweist, das dem eines Sprints entspricht. Die zusammenwachsenden Knochenfragmente wachsen somit unter Belastungsbedingungen zusammen, die bei dieser Person bei Ausübung des Sportes vorhanden sind, so daß nach dem Heilungsprozeß keine Anpassungsschwierigkeiten an diese Art von Belastungen bestehen. Es ist selbstverständlich möglich, dies an alle Sportarten anzupassen, d.h. beispielsweise bei Frakturen von Skifahrern, Radfahrern oder auch Dehnungsverletzungen von Ballsportlern, insbesondere im Bereich der Fußgelenke oder auch im Bereich der Schulter, wie beispielsweise bei Tennisspielern.

In einer besonderen Ausgestaltung der Erfindung sind beidseits der Läsion angeordnete Retentionsmittel vorgesehen, die derart miteinander verbunden sind, daß sie mittels des zumindest einen Antriebes hin- und herbewegbar sind, so daß die retenierten Körperteile die lädierten Bändern und/oder die lädierten Knochen in deren physiologischen Belastungsrichtungen bewegen.

Diese Maßnahme hat den Vorteil, daß durch die beidseits der Läsion angeordneten Retentionsmittel jegliche mögliche Relativbewegungen der beidseits der Läsion angeordneten Körperteile stimuliert werden können. Derartige Relativbewegungen können eine lineare Hin- und Herbewegung ein Verdrillen oder Verdrehen, ein gelenkartiges Abknicken oder auch Überlagerungen von derartigen Bewegungen sein. Es ist dann möglich, insbesondere bei Frakturen oder Bänderrißen oder Bänderdehnungen im Bereich von Gelenken, beispielsweise im Bereich des Fußgelenkes, einzelne Bewegungsabläufe oder auch überlagerte komplizierte Bewegungsabläufe während des Heilungsvorganges zu stimulieren. Oftmals sind Frakturen auch gleichzeitig mit Bänderrißen oder Bänderdehnungen verbunden. Durch die jetzt vorgeschlagene Regelung der Stimulationsamplitude und der -kraft kann dann in der jeweiligen Heilungsphase die optimale Stimulation von Bänder einerseits und zum Zusammenwachsen der Knochen andererseits, gesteuert werden.

In einer weiteren Ausgestaltung der Erfindung sind die Retentionsmittel als zweiteiliger Kunststoff- oder Gipsverband ausgebildet, an dessen beiden Teile jeweils Funktionsteile des Antriebes angebracht sind, so daß bei laufendem Antrieb die beiden Teile relativ zueinander hin- und herbewegbar sind.

Diese Maßnahme hat den Vorteil, daß die Vorrichtung integraler Bestandteil des Kunststoff- oder Gipsverbandes ist, so daß keine weiteren zusätzlichen Gerätschaften angebracht werden müssen. Der Kunststoff- oder Gipsverband dient somit als Träger und Widerlager der Vorrichtung.

Es ist dabei insbesondere von Vorteil, wenn zwischen den beiden Teilen zumindest ein Scharnier vorgesehen ist, so daß Läsionen im Gelenkbereich nicht nur linear hin- und herbewegbar, sondern auch gelenkig verschwenkend stimulierbar sind.

In einer weiteren Ausgestaltung der Erfindung liegt das Meßglied im Bereich der Läsion auf der Haut des Körperteiles.

Diese Maßnahme hat den Vorteil, daß ein auf der Haut aufliegendes Meßglied wenig störend für den Patienten und auch für den Heilungsprozeß ist. Die Anordnung im Läsionsbereich ermöglicht jedoch Messungen unmittelbar in dem Bereich, in dem der Heilungsprozeß vonstatten geht.

In einer weiteren Ausgestaltung besteht das Meßglied aus einem Ultraschallkopf mit zwei kreuzweise angeordneten Sendern/Empfängern von Ultraschallwellen, wodurch kontinuierliche Bewegungen im Läsionsbereich erfaßbar sind. Es ist auch möglich, das Meßglied aus einem hochauflösenden Ultraschallkopf aufzubauen, über den fortwährend das Maß der Bewegungen im Läsionsbereich erfaßt wird.

Diese Maßnahme hat den Vorteil, daß durch die für den Menschen schmerzlosen Ultraschallwellen eine schmerz- und störungsfreie Erfassung des Fortschreitens des Heilungsprozesses im Läsionsbereich durchgeführt werden kann.

In einer weiteren Ausgestaltung der Erfindung weist das Meßglied ein Piezoelement auf, das kontinuierliche Bewegungen im Läsionsbereich erfaßt und die erfaßten Meßwerte als elektrische Impulse an die Regeleinheit weitergibt.

In einer weiteren Ausgestaltung der Erfindung sind die Retentionsmittel als Fixateur Externe ausgebildet, der über ein Verstellelement verbundene, beidseits der Läsion angeordnete Halter aufweist, die über Stifte fest mit den Knochenbruchstücken verbunden sind, und das Meßglied ist am Fixateur oder am Verstellelement angeordnet und erfaßt deren Verformungen.

Diese Maßnahme hat den Vorteil, daß auf konstruktiv besonders einfache und meßtechnisch sehr zuverlässige Art und Weise das Fortschreiten des Heilungsprozesses erfaßt werden kann. Dadurch, daß die Stifte des Fixateurs Externs fest in den Knochenfragmenten aufgenommen sind, läßt das Maß der Widerstandskraft, die sich einem relativen Aufeinanderzubewegen der Halteteile entgegenstellt, einen Schluß auf das Fortschreiten des Heilungsprozesses zu. Ist das Regenerationsgewebe an den Bruchstellen zu Beginn eines Heilungsprozesses noch relativ weich, lassen sich die Halteteile des Fixateur Externes noch gegen eine relativ geringe Widerstandskraft hin- und herbewegen. Steigt diese Kraft an, ist dies ein Maß für eine verstärkte Knochenbildung, wobei die Widerstandskraft dann dazu herangezogen werden kann, eine entsprechende Stimulationskraft hervorzurufen.

Es ist weiter vorteilhaft, am Fixateur schlitten- oder teleskopartige Halter vorzusehen, und es ist ferner vorteilhaft, deren Verformungen über ein Meßglied, beispielsweise über zumindest einen Dehnungsstreifen zu erfassen.

Weitere Vorteile ergeben sich aus der Beschreibung und den beiliegenden Zeichnungen.

Es versteht sich, daß die vorstehend genannten und die nachstehend noch zu erläuternden Merkmale nicht nur in der jeweils angegebenen Kombination, sondern auch in anderen Kombinationen und in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschreiben und erläutert. Es zeigen:
- Fig. 1: einen Unterschenkel-Kunststoffverband mit einer erfindungsgemäßen Vorrichtung;
- Fig. 2: einen ausschnittsweisen vergrößerten Frontalschnitt längs der Linie II-II in Fig. 1, wobei ein Sprunggelenk mit einer Läsion (Verletzung) dargestellt ist;
- Fig. 3: einen Transversalschnitt längs der Linie III-III in Fig. 1;
- Fig. 4: ein weiteres Ausführungsbeispiel einer erfindungsgemäßen Vorrichtung zur stimulierenden rhytmischen Hin- und Herbewegung in Zusammenhang mit einem Kunststoff- oder Gipsverband;
- Fig. 5: ein Kugelgelenk, das zur Verwendung in Zusammenhang mit der in Fig. 4 dargestellten Vorrichtung vorgesehen ist;
- Fig. 6: stark schematisiert einen Regelkreis der erfindungsgemäßen Vorrichtung;
- Fig. 7: ein als Ultraschallkopf ausgebildetes Meßglied des Regelkreises von Fig. 6;
- Fig. 8: ein als Piezoelement ausgebildetes Meßglied des Regelkreises von Fig. 6;
- Fig. 9: ein weiteres Ausführungsbeispiel einer erfindungsgemäßen Vorrichtung in Zusammenhang mit einem Fixateur-Externe, und
- Fig. 10: ein Stimulations-Festigkeits-Diagramm der in Fig. 9 dargestellten Vorrichtung.

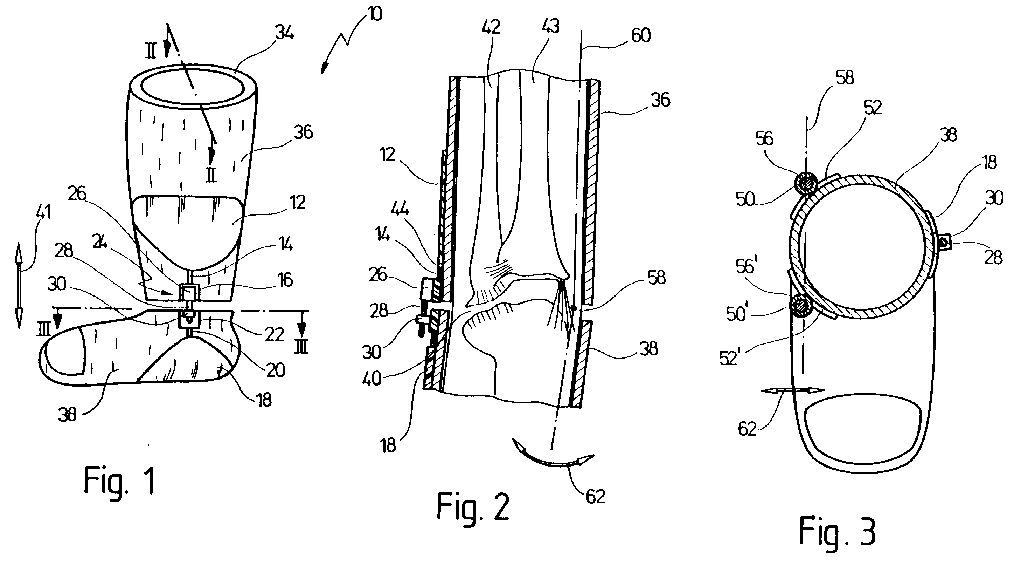

Eine in Fig. 1 und 2 dargestellte erfindungsgemäße Vorrichtung 10 weist eine erste, etwa dreieckförmige Platte 12 auf, von deren eine Dreiecksecke ein Stab 14 vorspringt, an dessen äußerem freien Ende ein Ansatz 16 vorgesehen ist. Spiegelbildlich dazu ist eine zweite Platte 18 vorgesehen, deren von einer Dreiecksecke vorspringender Stab 20 an seinem vorderen freien Ende mit einem Ansatz 22 versehen ist. Die beiden Ansätze 16 und 22 sind über einen Antrieb 24 verbunden.

Der Antrieb 24 weist einen am Ansatz 16 befestigten Motor 26 auf, dessen Antriebsspindel 28 in einer Gewindebuchse 30, die mit dem Ansatz 22 verbunden ist, eingedreht ist. Die Platte 12 ist auf die Außenseite eines oberen Teiles 36 eines Kunststoffverbandes 34 modelliert, und zwar derart, daß deren Ansatz 16 gerade am unteren abgeschnittenen Ende des oberen Teiles 36 endet. Die Platte 18 ist spiegelbildlich dazu derart im unteren Teil 38 des Kunststoffverbandes 34 angeordnet, daß deren Ansatz 22 fluchtend am oberen Ende des unteren Teiles 38 endet. Zwischen dem oberen Teil 36 und dem unteren Teil 38, die einen Abstand von etwa 2 cm aufweisen, ist die Läsion 40 gelegen (siehe insbesondere Fig. 2), die mittels des Kunststoffverbandes 34 gestützt werden soll.

Der zweiteilige Kunststoffverband 34 wird so hergestellt, daß zunächst ein einteiliger Verband in an sich bekannter Weise angebracht und aushärten gelassen wird. Anschließend wird der Kunststoffverband 34 mittels eines Sägeschnittes auf Höhe der Läsion 40 rundum aufgesägt, wobei die beiden Teile 36 und 38 entstehen. Anschließend werden die Platten 12 und 18 anmodelliert.

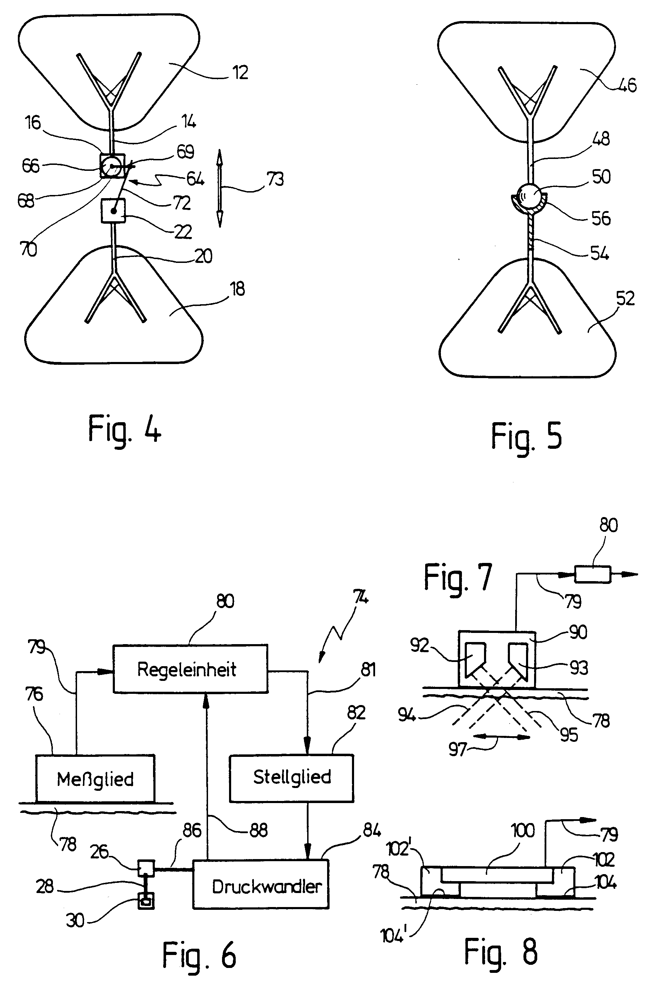

Der Motor 26 des Antriebes 24 wird derart betrieben, daß er die Spindel 28 hin- und herdreht, wodurch die beiden Teile 36, 38 des Kunststoffverbandes 34 aufeinander zu bzw. voneinander weg bewegt werden, wie dies in Fig. 1 durch einen Pfeil 41 dargestellt ist. Da die beiden Teile 36 und 38 des Kunststoffverbandes 34 beidseits einer Läsion 40 zum Liegen kommen und außerdem an dem Körperteil, hier dem Bein und dem Fuß, fest anliegen, werden die in den Körperteilen eingebetteten Knochen ebenfalls aufeinander zu bzw. voneinander weg bewegt. Dadurch wird der Heilungsvorgang im Bereich der Läsion beschleunigt. In einer ersten Heilungsphase wird der Motor 26 derart betrieben, daß im Gewebe im Bereich der Läsion Bewegungen mit Stimulationsamplituden im Bereich von etwa 0,1 bis 1 mm stimuliert werden und zwar mit einer Frequenz von 0,5 bis 5 Hz. Dabei finden etwa 1 bis 5 Stimulationszyklen pro Tag und zwar jeweils mit einer Dauer von etwa 15 Minuten statt. Die Stimulationsstärke liegt dabei im Bereich von 20 bis 800 N. Die Regelung der Stimulation wird später in Zusammenhang mit der Fig. 6 näher beschrieben. Dazu ist der in Fig. 1 dargestellte Antrieb 24 mit dem in Fig. 6 dargestellten Regelkreis 74 verbunden.

Soll die in Fig. 1 durch den Pfeil 41 angezeigte Stimulationsrichtung ausschließlich erzeugt werden, so kann es ausreichend sein, lediglich den dargestellten Antrieb 24 vorzusehen.

Es ist auch möglich, auf der gegenüberliegenden Außenseite des Kunststoffverbandes 34 spiegelbildlich nochmals eine derartige Vorrichtung vorzusehen.

Ist es wünschenswert, nicht nur eine lineare Hin- und Herbewegung zu erreichen, wie dies in Fig. 1 durch den Pfeil 41 dargestellt ist, sondern auch ein seitliches Abkippen des unteren Teiles 38 erreicht werden, wie dies in Fig. 2 und 3 dargestellt ist, sind noch zusätzliche Scharniergelenke vorgesehen, wie sie in Fig. 5 dargestellt sind.

Ein solches Scharniergelenk weist eine erste Platte 46 auf, die gleichermaßen wie die Platte 12 oder 18 ausgebildet ist. Der sich von der Platte 46 forterstreckende Stab 48 ist an seinem äußeren Ende mit einer Kugel 50 versehen.

Die Kugel 50 ist in einer Pfanne 56 aufgenommen, die am äußeren Ende eines Stabes 54 angeordnet ist, der mit einer Platte 52 verbunden ist.

Wie insbesondere aus Fig. 3 zu entnehmen, sind etwa umfänglich um 120° versetzt zwei solche Scharniere vorgesehen, wobei jeweils die untere Platte 52 bzw. 52', die die Pfanne 56 bzw. 56' trägt, an der Außenseite des unteren Teiles 38 des Kunststoffverbandes 34, der das Fußteil darstellt, anmodelliert sind. In den Pfannen 56 bzw. 56' sind dann die Kugeln 50 bzw. 50' der am oberen Teil 36 befestigten Platten aufgenommen.

Dadurch ist es dann möglich, daß das untere Teil 38 des Kunststoffverbandes 34 seitlich relativ zum oberen Teil 36 verschwenkt werden kann, wie dies in Fig. 2 und 3 durch einen Doppelpfeil 62 dargestellt ist.

Da bei dieser Schwenkbewegung sich auch der Antrieb 24, d.h. der Motor 26, die Spindel 28 und die darin aufgenommene Buchse 30 verschwenkt, ist in diesem Fall der Stab 14 über ein Gelenk 44 mit der Platte 12 verbunden.

Dadurch, daß das untere Teil 38 um eine Achse 58 verschwenkbar ist, kann bereits während des Heilungsprozesses eine Bewegung stimuliert werden, die einem späteren Bewegungsablauf zwischen Fuß und Beinteil entspricht. Es ist selbstverständlich möglich, auch Bewegungsabläufe vorzusehen, die ein Abkippen des Fußteiles nach vorne bzw. unten ermöglicht. Die häufigsten Verletzungen finden jedoch an den seitlichen Bändern statt, so daß die in Fig. 2 und 3 dargestellte Verschwenkmöglichkeit einen häufigen Anwendungsfall darstellt.

In Fig. 4 ist ein weiteres Ausführungsbeispiel eines Antriebes 64 dargestellt.

Der Antrieb 64 weist einen Motor 66 auf, der auf dem Ansatz 16, der mit der Platte 12 verbunden ist, montiert ist. Die Welle 68 des Motors 66 ist mit einem seitlich vorspringenden Exzenter 70 versehen, der als Stab ausgebildet ist. Der Exzenter 70 ist über ein Gelenk 69 mit einer Pleuelstange 72 verbunden, die gelenkig am Ansatz 22 der Platte 18 angebracht ist. Durch Verschieben des Gelenkes 69 längs des stabförmigen Exzenters 70, kann die Exzentrizität geregelt bzw. gesteuert werden, so daß die durch den Doppelpfeil 73 angedeutete Relativbewegung zwischen Platte 12 und 18 steuerbar ist. Dazu ist dann der Antrieb 64 ebenfalls beispielsweise mit einem in Fig. 6 dargestellten Regelkreis 74 verbunden.

Der Regelkreis 74 weist ein Meßglied 76 auf, das auf der Haut 78 des Körperteiles angebracht ist, an dem die Läsion vorhanden ist, und zwar in möglichst unmittelbarer Nähe des Läsionsortes. Es ist beispielsweise vorgesehen, das Meßglied 76 in dem Spalt zwischen dem oberen 36 und unteren Teil 38 des Kunststoffverbandes 34 (siehe Fig. 1) anzuordnen.

Das Meßglied 76 ist über eine Leitung 79 mit einer Regeleinheit 80 verbunden, die wiederum über eine Leitung 81 mit einem Stellglied 82 verbunden ist. Das Stellglied 82 ist mit einem Druckwandler 84 verbunden, der wiederum über eine Leitung 86 mit dem Motor 26 oder beispielsweise mit dem Motor 66 verbunden ist. Der Druckwandler 84 ist ferner über eine Leitung 88 mit der Regeleinheit 80 verbunden.

Das Stellglied 82 bewirkt z.B. eine Verstellung des Motores 26 im Hinblick auf die Frequenz und Größe der Drehzyklen.

In Fig. 7 ist ein Meßglied 76 dargestellt, das als Ultraschallkopf 90 ausgestaltet ist. Der Ultraschallkopf 90 ist mit zwei Sendern/Empfängern 92, 93 versehen, die gekreuzte Ultraschallwellen 94, 95 aussenden. Durch die ausgesandten bzw. erfaßten reflektierten Ultraschallwellen können Bewegungen im daruntergelegenen Gewebe, wie sie durch einen Doppelpfeil 97 angedeutet sind, mit einer Genauigkeit von 0,1 ± 0,05 mm registriert werden. Der Ultraschallkopf 90 liegt dabei in einer gelgefüllten Kunststoffhaube auf der Haut 78 auf.

In Fig. 8 ist ein weiteres Ausführungsbeispiel eines Meßgliedes dargestellt, das als Piezoelement 100 ausgebildet ist.

Das Piezoelement 100 ist in elastische Kunststoffhalterungen 102, 102' eingebettet, deren Unterseiten mit Klebeflächen 104, 104' versehen sind, über die sie auf der Haut 78 fixiert werden können. Durch Bewegungen im Gewebe spricht das Piezoelement 101 an und gibt ein Maß für den Widerstand im Gewebe.

Der in Fig. 6 dargestellte Druckwandler 84 dient dazu, um während eines Stimulationszyklus der Belastung ein bestimmtes Kraft-Zeitprofil zu geben, wie dies eingangs erwähnt wurde.

Dadurch ist es dann möglich, beispielsweise während eines Stimulationsvorganges einen Sprint oder dgl. zu simulieren.

In einem weiteren, hier nicht dargestellten, Ausführungsbeispiel wird die Stimulationsbewegung durch einen Muskelstimulator erzeugt. Es ist insbesondere bei Hochleistungssportlern bekannt geworden, um während eines Heilungsvorganges die Muskelmasse aufrecht zu erhalten, diese durch einen Muskelstimulator zu Kontraktionen anzuregen. Bei diesen Kontraktionen kann durch entsprechende Ausgestaltung des Gips- oder Kunststoffverbandes, beispielsweise wie in Fig. 1 dargestellt, die Möglichkeit eröffnet werden, daß dadurch auch eine stimulierte Bewegung der Knochen oder Bänder stattfindet. Es ist dann lediglich noch ein Meßglied 76 vorzusehen, das den Heilungsverlauf registriert, woraufhin dann über einen Regelkreis das Muskelstimulationsgerät gesteuert wird.

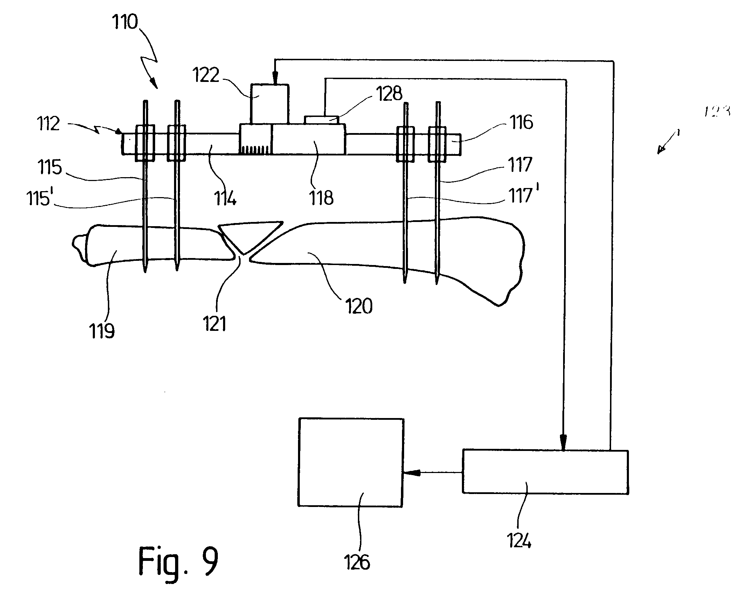

Bei einer in Fig. 9 dargestellten weiteren Ausführung einer erfindungsgemäßen Vorrichtung 110 ist diese als Bestandteil eines Fixateur Externes 112 ausgebildet.

Der Fixateur Externe 112 weist einen ersten Halter 114 auf, der mit Stiften 115, 115' versehen ist. Der Halter 114 ist über ein Verstellelement 118 mit einem zweiten Halter 116 verbunden, der mit Stiften 117, 117' versehen ist. Die Stifte 115, 115' stecken in einem Knochenstück 119, wohingegen die Stifte 117, 117' in einem zweiten Knochenstück 120 stecken.

Zwischen den Knochenstücken 119 und 120 ist eine Läsion 121 in Form eines komplizierten Knochenbruches, bei dem ein hier nicht näher bezeichnetes, weiteres, etwa dreieckförmiges Knochenbruchstück vorhanden ist. Auf dem Verstellelement 118 ist ein Motor 122 vorgesehen, der eine rhytmische Hin- und Herbewegung der beiden Halter 114, 116 verursacht. Dazu können diese teleskopartig ineinander verschiebbar sein.

Der Motor 122 ist Teil eines Regelkreises 123. Der Motor 122 dient als Stellglied und ist mit einer Regeleinheit 124 verbunden, die wiederum mit einem Monitor 126 verbunden ist. Die Regelstrecke liegt in der Läsionszone 121. Ein Meßaufnehmer 128 ist am Fixateur 112, in diesem Fall am Verstellelement 118, angebracht und kann Signale an die Regeleinheit 124 geben. Das Meßglied 128 erfaßt diejenige Widerstandskraft, die zwischen den Haltern 114 und 116 vorhanden ist, falls diese aufeinander zu bewegt oder gegeneinander verdrillt oder verbogen werden. Die zunehmende Widerstandskraft ist ein Maß für das Fortschreiten des Heilungsprozesses an der Läsion 121.

Der Meßaufnehmer 128 erfaßt kleinste Längen- oder Wegänderungen in oder am Fixateur 112 und kann somit die Widerstandskraft indirekt erfassen. Gemessen werden können diese minimalen Bewegungen mit Dehnungsmeßstreifen, oder mit DC/DC Wegsensoren, oder mit induktiven Wegsensoren oder auf Piezobasis. Mehrere Dehnungsmeßstreifen können in verschiedenen Richtungen geschützt im Fixateur 112 angebracht sein und so gleichzeitig ein Verdrillen oder Verbiegen des Fixateurs 112 erfassen.

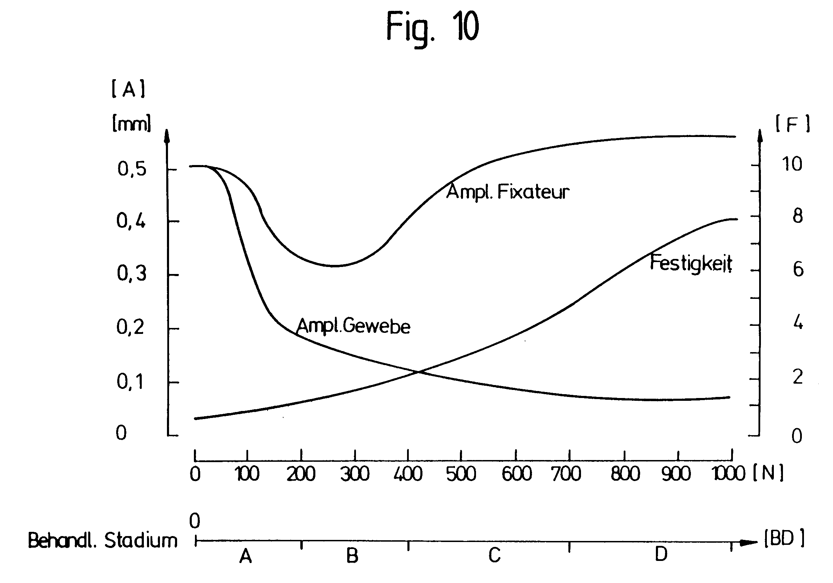

Der Regelkreis 123, der mikroprozessorgesteuert ist, steuert den Fixateur Externe 112 derart, daß das in Fig. 10 dargestellte Stimulations-Festigkeitsdiagramm resultiert. Die Stimulationsamplitude A, die im Diagramm in Millimeter aufgetragen ist, betrifft die Amplitude mit der die Halter in Längsrichtung der Knochenstücke 119, 120 bzw. in Längsrichtung des Fixateurs 112 hin- und herbewegt werden (Ampl. Fixateur) bzw. die im Gewebe erzeugten Stimulationsamplituden (Ampl. Gewebe). Die Festigkeitskurve betrifft die relative Festigkeit F zwischen den an der Läsion 121 zusammenwachsenden Knochen 119, 120 im Laufe eines Behandlungszeitraumes, der durch die Behandlungsstadien (BD), A, B, C und D gekennzeichnet ist.

Wie aus Fig. 10 zu entnehmen, wird in einem ersten Behandlungsstadium A ausgehend von einer maximalen Stimulationsamplitude im Gewebe von 0,5 mm diese sehr rasch auf eine Stimulationsamplitude von 0,2 mm herabgesetzt. Im Diagramm von Fig. 10 betrifft die obere Kurve den Amplitudenverlauf des Fixateur Externes 112, die darunterliegende Kurve den Amplitudenverlauf im Gewebe, also im Bereich der Läsion 121. Zu Beginn des Behandlungsstadiums A, d.h. die Knochenfragmente 119, 120 sind noch ohne Verbindung, ist die Amplitude des Fixateurs gleich der Amplitude des Gewebes. Mit zunehmender Festigkeit verursacht eine bestimmte Amplitude des Fixateurs eine geringere Stimulationsamplitude im Gewebe. Im Laufe des Behandlungsstadiums A wird die Stimulationskraft etwa linear auf 200 N angehoben. In einem zweiten Behandlungsstadium B durchläuft die Amplitudenkurve des Fixateurs eine Mulde, in der bei steigender Stimulationskraft nur eine geringe Abnahme der Stimulationsamplitude im Gewebe erfolgt. In den weiteren Behandlungsstadien C und D, die mit steigender Stimulationskraft bis zu 1000 N stattfinden, nimmt die Festigkeit bis auf einen Relativendwert von 8 zu, der bereits einem sehr fortgeschrittenen Heilungsstadium des Knochenbruches entspricht. Die Amplitude am Fixateur Externe muß dann wieder den maximalen Ausgangswert von 0,5 mm aufweisen, um im Gewebe noch eine Stimulationsamplitude von 0,1 mm zu erreichen.

Bei einem in Fig. 10 dargestellten vorbestimmten Stimulations-Festigkeitsdiagramm kann eine erhebliche Verkürzung der Heilungszeit, zumindest um mehrere Tage, wenn nicht gar Wochen, gegenüber einem nicht geregelten Stimulationsprogramm erreicht werden.

## Patentansprüche

1. Vorrichtung zur Retention von Körperteilen mit lädierten Bändern und/oder lädierten Knochen (119, 120), mit zumindest einem Antrieb (24, 64) zum stimulierenden rhythmischen Hin- und Herbewegen der retentierten Körperteile im Läsionsbereich (40, 121) mit einer bestimmten Stimulationsamplitude und einer bestimmten Stimulationskraft, gekennzeichnet durch einen Regelkreis (74, 123) mit einem Meßglied (76, 128), das die Festigkeit im Läsionsbereich (40, 121) erfaßt, mit einer Regeleinheit (80), die die vom Meßglied (76, 128) erfaßten Festigkeitsdaten als Basis für ein Regelsignal heranzieht, und mit einem Stellglied (82), das die Stimulationsamplitude und die Stimulationskraft während eines Heilungsvorganges in Abhängigkeit vom Regelsignal einstellt.

2. Vorrichtung nach Anspruch 1, dadurch gekennzeichnet, daß die Regeleinheit (80) eine solche Kennlinie aufweist, daß bei zunehmender Festigkeitsentwicklung von lädierten Knochen (119, 120) mit geringer werdender Stimulationsamplitude und/oder mit zunehmender Stimulationskraft stimuliert wird.

3. Vorrichtung nach Anspruch 2, dadurch gekennzeichnet, daß ein Kraftwandler (84) vorgesehen ist, der die Stimulationskraft während eines Stimulationsvorganges mit einem Kraft-Zeit-Profil stimuliert, wobei das Kraft-Zeit-Profil einem typischen Bewegungsablauf der retentierten Körperteile entspricht.

4. Vorrichtung nach einem der Ansprüche 1 bis 3, dadurch gekennzeichnet, daß beidseits der Läsion (40, 121) angeordnete Retentionsmittel (34, 112) vorgesehen sind, die so miteinander verbunden sind, daß sie mittels des zumindest einen Antriebes (24, 64) derart hin- und herbewegbar sind, daß die retentierten Körperteile die lädierten Bänder und/oder die lädierten Knochen (119, 120) in deren physiologischen Belastungsrichtungen bewegen.

5. Vorrichtung nach Anspruch 4, dadurch gekennzeichnet, daß die Retentionsmittel als zweiteiliger Kunststoff- oder Gipsverband (34) ausgebildet sind, an dessen beiden Teilen (36, 38) jeweils Funktionsteile (26, 28; 66, 68, 69, 70, 72) des Antriebes (24, 64) angebracht sind, so daß bei laufendem Antrieb (24, 64) die beiden Teile (36, 38) relativ zueinander hin- und herbewegbar sind.

6. Vorrichtung nach Anspruch 5, dadurch gekennzeichnet, daß zwischen den beiden Teilen (36, 38) zumindest ein Scharnier vorgesehen ist, so daß Läsionen (40) in Gelenkbereichen nicht nur linear hin- und herbewegbar sind, sondern auch gelenkig verschwenkend stimulierbar sind.

7. Vorrichtung nach einem der Ansprüche 1 bis 6, dadurch gekennzeichnet, daß das Meßglied (76) im Bereich der Läsion (40) auf der Haut (78) des Körperteiles liegt.

8. Vorrichtung nach Anspruch 6, dadurch gekennzeichnet, daß das Meßglied einen Ultraschallkopf (90) mit zwei kreuzweise angeordneten Sendern/Empfängern (92, 93) von Ultraschallwellen (94, 95) aufweist, um fortwährend Bewegungen im Läsionsbereich (40) zu erfassen.

9. Vorrichtung nach Anspruch 6, dadurch gekennzeichnet, daß das Meßglied aus einem hochauflösenden Ultraschallkopf besteht, der lädierte Bandstrukturen erfassen kann und in Kombination mit einem Rechner ein Bild mit zwei- oder dreidimensionaler Darstellung der Bänder erzeugen kann und fortwährend das Maß der Bewegungen im Läsionsbereich erfaßt.

10. Vorrichtung nach Anspruch 7, dadurch gekennzeichnet, daß das Meßglied ein Piezoelement (100) aufweist, das kontinuierliche Bewegungen im Läsionsbereich (40) erfaßt und diese in Form von elektrischen Impulsen an die Regeleinheit (80) weitergibt.

11. Vorrichtung nach Anspruch 10, dadurch gekennzeichnet, daß das Piezoelement (100) von zumindest zwei verformbaren Haltern (102, 102'), insbesondere aus Kunststoffmaterial, aufgenommen ist, die auf der Haut (78) des Körperteils aufgeklebt sind.

12. Vorrichtung nach Anspruch 4, dadurch gekennzeichnet, daß die Retentionsmittel als Fixateur Externe (112) ausgebildet sind, der über ein Verstellelement (118) verbundene, beidseits der Läsion (121) angeordnete Halter (114, 116) aufweist, die über Stifte (115, 115'; 117, 117') fest mit den Knochenbruchstücken (119, 120) verbunden sind, und daß das Meßglied (128) am Fixateur angeordnet ist, und Verformungen des Fixateurs erfaßt.

13. Vorrichtung nach Anspruch 12, dadurch gekennzeichnet, daß die Halter (114, 116) schlitten- oder teleskopartig bewegbar sind.

14. Vorrichtung nach Anspruch 12 oder 13, dadurch gekennzeichnet, daß das Meßglied am Verstellelement (118) angeordnet ist.

15. Vorrichtung nach Anspruch 13, dadurch gekennzeichnet, daß das Meßglied an einem der teleskopartigen Halter (114, 116) angeordnet ist, und dessen Verformung erfaßt.

16. Vorrichtung nach einem der Ansprüche 1 bis 4, dadurch gekennzeichnet, daß als Antrieb eine Vorrichtung zur elektrischen Stimulation von Muskeln vorgesehen ist, welche die Muskeln der Körperteile im Läsionsbereich in physiologischen Bewegungsrichtungen der Körperteile stimuliert.

17. Verfahren zur Retention von Körperteilen mit lädierten Bändern und/oder lädierten Knochen (119, 120), wobei durch rhythmisches Hin- und Herbewegen der retentierten Körperteile im Läsionsbereich (40, 121) mit einer bestimmten Stimulationsamplitude und einer bestimmten Stimulationskraft stimuliert wird, dadurch gekennzeichnet, daß die Festigkeitsentwicklung im Läsionsbereich gemessen wird, daß daraus ein Regelsignal bestimmt wird, und daß in Abhängigkeit der erfaßten Daten der Festigkeitsentwicklung die Stimulationskraft und die Stimulationsamplitude geregelt wird.

18. Verfahren nach Anspruch 17, dadurch gekennzeichnet, daß die Stimulation so geregelt wird, daS bei zunehmender Festigkeitsentwicklung von lädierten Knochen (119, 120) mit geringer werdender Stimulationsamplitude stimuliert wird.

19. Verfahren nach Anspruch 17 oder 18, dadurch gekennzeichnet, daß bei zunehmender Festigkeitsentwicklung von lädierten Knochen (119, 120) mit zunehmender Stimulationskraft stimuliert wird.

20. Verfahren nach einem der Ansprüche 17 bis 19, dadurch gekennzeichnet, daß die Stimulationskraft mit einem Kraft-Zeit-Profil stimuliert wird, das einem Kraft-Zeit-Profil eines typischen Bewegungsablaufs der retentierten Körperteile entspricht.

## Claims

1. Apparatus for the retention of body parts with injured ligaments and/or injured bones (119, 120) having at least one drive unit (24, 64) for producing stimulating, rhythmical reciprocating movement of the retained parts of the body in the injured area (40, 121) by means of a defined stimulation amplitude and a defined stimulation power, characterised by a feedback control system (74, 123) with a measuring component (76, 128) that scans the degree of stability within the injured area (40, 121), a regulating unit (80) that uses the stability data ascertained by the measuring component (76, 128) as the basis for a regulating signal, and a control element (82) that is dependent on the regulating signal in order to adjust the stimulation amplitude and the stimulation power during a course of healing.

2. Apparatus according to claim 1, characterised in that the regulating unit (80) displays a characteristic line of such a nature that, with increasing stability development of the injured bones (119, 120), stimulation is applied with decreasing stimulation amplitude and/or with increasing stimulation power.

3. Apparatus according to claim 2, characterised in that a power transformer (84) is provided which stimulates the stimulation power in the course of a stimulation process by means of a power-time-profile, wherein the power-time-profile corresponds to a typical sequence of movement of the retained parts of the body.

4. Apparatus according to one of the claims 1 to 3, characterised in that on both sides of the injured area (40, 121) retaining devices (34, 112) are arranged which are connected to each other in such a way that they can be moved to and fro by means of the at least one drive unit (24, 64) with the result that the retained parts of the body move the injured ligaments and/or the injured bones (119, 120) within their physiological load directions.

5. Apparatus according to claim 4, characterised in that the retaining devices are constructed as a two-part synthetic or plaster cast (34) on the two respective sections of which (36, 38) are attached functional parts (26, 28; 66, 68, 69, 70, 72) of the drive unit (24, 64) so that, when the drive unit (24, 64) is operating, the two sections (36, 38) can be moved to and fro relative to each other.

6. Apparatus according to claim 5, characterised in that, between the two sections (36, 38), at least one articulation element is provided so that injured areas (40) in the region of joints of the body can not only be moved to and fro in a linear plane but can also be stimulated by flexible pivoting.

7. Apparatus according to one of the claims 1 to 6, characterised in that the measuring component (76) rests upon the skin surface (78) of the part of the body in the region of the injury (40).

8. Apparatus according to claim 7, characterised in that the measuring component comprises an ultrasonic head (90) with two diagonally arranged transmitters/receivers (92, 93) of ultrasonic waves (94, 95) in order to provide continuous scanning of movements within the injured area (40).

9. A device according to claim 7, characterised in that the measuring component consists of a high resolution ultrasonic head that is capable of scanning injured ligamental structures and, in conjunction with a computer, can produce a picture showing either a two- or three-dimensional image of the ligaments and will provide continuous scanning of the extent of movement within the injured area.

10. Apparatus according to claim 7, characterised in that the measuring component comprises a piezoelectric element (100) that scans continuous movements in the injured area (40) and transmits these to the regulating unit (80) in the form of electrical impulses.

11. A device according to claim 10, characterised in that the piezoelectric element (100) is held in position by at least two deformable holders (102, 102') preferably constructed of a synthetic material and glued to the surface of the skin (78) of the relevant part of the body.

12. Apparatus according to claim 4, characterised in that the retaining devices are constructed as a fixateur externe (112) comprising holders (114, 116) located to either side of the injury (121) and connected to each other by means of an adjusting element (118), said holders (114, 116) being connected rigidly to the broken pieces of bone (119, 120) by means of pins (115, 115'; 117, 117'), and that the measuring component (128) is arranged on the fixateur and detects any deformations of the fixateur.

13. Apparatus according to claim 12, characterised in that the holders (114, 116) are capable of either sliding or telescopic movement.

14. Apparatus according to claim 12 or 13, characterised in that the measuring component is arranged on the adjusting element (118).

15. Apparatus according to claim 13, characterised in that the measuring component is arranged on one of the telescopic holders (114, 116) and detects any deformations of the latter.

16. Apparatus according to one of the claims 1 to 4, characterised in that, as a drive unit, a device suitable for the electrical stimulation of muscles is provided which stimulates the muscles of the parts of the body in the injured area in the physiological direction of movement of those same parts of the body.

17. A method of the retention of parts of the body with injured ligaments and/or injured bones (119, 120), wherein, by means of rhythmical reciprocating movement of the retained parts of the body within the injured area (40, 121), stimulation is provided with a defined stimulation amplitude and a defined stimulation power, characterised in that the stability development in the injured area is measured, that a regulating signal is determined from this and that the stimulation power and the stimulation amplitude are regulated dependent upon the data obtained from the stability development.

18. A method according to claim 17, characterised in that the stimulation is regulated in such a way that, with increasing stability development of the injured bones (119, 120), stimulation is applied with decreasing stimulation amplitude.

19. A method according to claim 17 or 18, characterised in that, with increasing stability development of the injured bones (119, 120), stimulation is applied with increasing stimulation power.

20. A method according to one of the claims 17 to 19, characterised in that the stimulation power is applied by means of a power-time-profile corresponding to a power-time-profile of a typical sequence of movement of the retained parts of the body.

## Revendications

1. Dispositif de maintien de parties du corps présentant des ligaments lésés et/ou des os lésés (119, 120), comportant dans la zone des lésions (40, 121) au moins un mécanisme d'entraînement (24, 64) pour le mouvement de va-et-vient rythmique stimulant de la partie du corps maintenue, à une amplitude de stimulation déterminée et à une force de stimulation déterminée, caractérisé par un circuit de régulation (74, 123) comportant un organe de mesure (76, 128) qui détecte la résistance de la zone des lésions (40, 121), comportant une unité de régulation (80) qui consulte les données de résistance détectées par l'organe de mesure (76, 128) comme base pour un signal de régulation, et comportant un organe de réglage (82) qui règle l'amplitude de stimulation et la force de stimulation au cours d'un processus de guérison en fonction du signal de régulation.

2. Dispositif selon la revendication 1, caractérisé en ce que l'unité de régulation (80) présente une courbe caractéristique telle que la stimulation se fait à une amplitude de stimulation devenant plus faible et/ou à une force de stimulation croissante, lors de l'augmentation de la résistance des os lésés (119, 120).

3. Dispositif selon la revendication 2, caractérisé en ce qu'est prévu un transducteur (84), qui stimule la force de stimulation pendant un processus de stimulation au moyen d'un profil force-temps, le profil force-temps correspondant au déroulement d'un mouvement typique de la partie du corps maintenue.

4. Dispositif selon l'une des revendications 1 à 3, caractérisé en ce que sont prévus des moyens de maintien (34, 112) disposés de part et d'autre de la lésion (40, 121) et raccordés les uns aux autres de manière à pouvoir effectuer un mouvement de va-et-vient au moyen d'au moins un mécanisme d'entraînement (24, 64), de telle façon que les parties du corps maintenues déplacent les ligaments et/ou os lésés dans leurs sens de mouvement physiologique.

5. Dispositif selon la revendication 4, caractérisé en ce que les moyens de maintien sont constitués sous la forme d'un bandage (34) en matière plastique ou en plâtre en deux parties, en ce que sur chacune de ces deux parties (36, 38) sont montés des éléments fonctionnels (26, 28 ; 66, 68, 69, 70, 72) du mécanisme d'entraînement (24, 64), de façon à ce que lorsque le mécanisme d'entraînement (24, 64) fonctionne, les deux parties (36, 38) peuvent effectuer un mouvement de va-et-vient l'une par rapport à l'autre.

6. Dispositif selon la revendication 5, caractérisé en ce qu'est prévu entre les deux parties (36, 38) au moins une charnière, de façon à ce que les lésions (40) dans les zones d'articulation ne puissent pas effectuer uniquement un mouvement de va-et-vient linéaire, mais puissent également être stimulées horizontalement de façon articulée.

7. Dispositif selon l'une des revendications 1 à 6, caractérisé en ce que l'organe de mesure (76) se trouve dans la zone de la lésion (40) sur la peau (78) de la partie du corps.

8. Dispositif selon la revendication 6, caractérisé en ce que l'organe de mesure présente une tête à ultrasons (90) comportant deux émetteurs/récepteurs (92, 93) d'ondes ultrasonores (94, 95) disposés en croix, pour détecter en continu les mouvements dans la zone des lésions (40).

9. Dispositif selon la revendication 6, caractérisé en ce que l'organe de mesure consiste en une tête à ultrasons haute résolution, qui peut détecter les structures de ligament lésées, et produire en association avec un calculateur une image des ligaments en représentation en deux ou en trois dimensions, et détecter en continu l'ampleur des mouvements dans la zone des lésions.

10. Dispositif selon la revendication 7, caractérisé en ce que l'organe de mesure présente un élément piézo-électrique (100), qui détecte en continu les mouvements dans la zone des lésions (40), et les transmet sous forme d'impulsions électriques à l'unité de régulation (80).

11. Dispositif selon la revendication 10, caractérisé en ce que l'élément piézo-électrique (100) repose sur au moins deux supports déformables (102, 102'), en particulier en matière plastique, qui sont collés sur la peau (78) de la partie du corps.

12. Dispositif selon la revendication 4, caractérisé en ce que les moyens de maintien sont conçus sous la forme d'un fixateur externe (112), qui présente des supports (114, 116) disposés de part et d'autre de la lésion (121), raccordés par un élément de réglage (118), qui sont raccordés de façon fixe par des chevilles (115, 115' ; 117, 117') aux fragments d'os (119, 120), et en ce que l'organe de mesure (128) est disposé au niveau du fixateur, et détecte les déformations du fixateur.

13. Dispositif selon la revendication 12, caractérisé en ce que les supports (114, 115) sont mobiles de façon coulissante ou télescopique.

14. Dispositif selon les revendications 12 ou 13, caractérisé en ce que l'organe de mesure est disposé au niveau de l'élément de réglage (118).

15. Dispositif selon la revendication 13, caractérisé en ce que l'organe de mesure est disposé au niveau des supports télescopiques (114, 116), et en détecte la déformation.

16. Dispositif selon l'une des revendications 1 à 4, caractérisé en ce qu'est prévu comme mécanisme d'entraînement un dispositif de stimulation électrique des muscles, qui stimule les muscles de la partie du corps dans la zone des lésions dans le sens de mouvement physiologique de la partie du corps.

17. Procédé de maintien de parties du corps présentant des ligaments lésés et/ou des os lésés (119, 120), une stimulation étant effectuée dans la zone des lésions (40, 121) par un mouvement de va-et-vient rythmique de la partie du corps maintenue, à une amplitude de stimulation déterminée et à une force de stimulation déterminée, caractérisé en ce que l'on mesure l'évolution de la résistance dans la zone des lésions, en ce que l'on détermine à partir de là un signal de régulation, et en ce que la force de stimulation et l'amplitude de stimulation sont régulées en fonction des données détectées d'évolution de la résistance.

18. Procédé selon la revendication 17, caractérisé en ce que la stimulation est régulée de façon à ce que la stimulation se fasse à une amplitude de stimulation devenant plus faible lors de l'augmentation de la résistance des os lésés (119, 120).

19. Procédé selon les revendications 17 ou 18, caractérisé en ce que la stimulation se fait à une force de stimulation croissante lors de l'augmentation de la résistance des os lésés (119, 120).

20. Procédé selon l'une des revendications 17 à 19, caractérisé en ce que la force de stimulation est stimulée par un profil force-temps, qui correspond à un profil force-temps du déroulement d'un mouvement typique de la partie du corps maintenue.
